(19) Europäisches Patentamt
European Patent Office
Office européen des brevets

(11) Veröffentlichungsnummer: **0 131 790**
**A2**

(12) # EUROPÄISCHE PATENTANMELDUNG

(21) Anmeldenummer: **84107251.5**

(22) Anmeldetag: **25.06.84**

(51) Int. Cl.[4]: **A 61 K 31/245**
A 61 K 47/00

(30) Priorität: **01.07.83 DE 3323833**

(43) Veröffentlichungstag der Anmeldung:
**23.01.85 Patentblatt 85/4**

(84) Benannte Vertragsstaaten:
**AT BE CH DE FR GB IT LI LU NL SE**

(71) Anmelder: **Troponwerke GmbH & Co. KG**
**Berliner Strasse 156**
**D-5000 Köln 80(DE)**

(72) Erfinder: **Dell, Hans-Dieter, Dr.**
**Kalmuentener Strasse 5**
**D-5060 Bergisch-Gladbach 2(DE)**

(72) Erfinder: **Kraus, Reinhold**
**Paffendorfstrasse 77**
**D-5000 Koeln 91(DE)**

(72) Erfinder: **Schierstedt, Detlef, Dr.**
**Henri-Dumont-Strasse 2**
**D-5205 St. Augustin 3(DE)**

(74) Vertreter: **Adrian, Albert, Dr. et al,**
**c/o BAYER AG Konzernverwaltung RP Patentabteilung**
**D-5090 Leverkusen 1, Bayerwerk(DE)**

(54) **Etofenamat-Zubereitung.**

(57) Die vorliegende Erfindung betrifft eine topisch anzuwendende, entzündungshemmende und analgetisch wirkende Arzneimittelzubereitung, welche als Wirkstoff Etofenamat und ferner ein oder mehrere Adjuvantien aus der Gruppe:
- Triglyceride,
- Ester ein- und/oder mehrwertiger Alkohole,
- höhere Alkohole

enthält.

EP 0 131 790 A2

TROPONWERKE GmbH & Co. KG

5000 Köln 80
Je/Ke-c

Etofenamat-Zubereitung

Die vorliegende Erfidnung betrifft eine topisch anzuwendende, entzündungshemmende und analgetisch wirkende Arzneimittelzubereitung, welche als wesentlichen Wirkstoff 2-(2-Hydroxyethoxyethyl-N-(α,α,α-trifluor-m-tolyl)anthranilat, nachfolgend Etofenamat genannt, enthält. Etofenamat besitzt folgende Struktur:

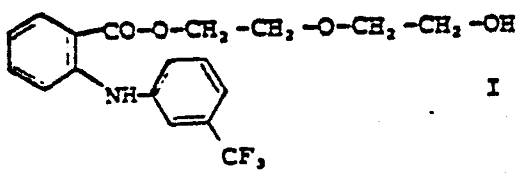

I

Etofenamat ist bereits als nichtsteroidales Arzneimittel mit guter entzündungshemmender Wirkung und hervorragender Verträglichkeit bekannt, vgl. Arzneimittelforschung 27 (I) Sonderheft 6 b, 1299 - 1364, 1977. Etofenamat wird bisher in Form alkoholhaltiger Gele eingesetzt. Durch den Alkohol wirkt das Gel beim Auftragen kühlend, was nicht von allen Patienten als angenehm empfunden wird. Ferner kann ein solches Gel nur bei unversehrter Haut aufgetragen werden, eine wünschenswerte Applikation bei Traumen mit offenen Wunden entfällt daher. Auch ist bei Patienten mit ausgesprochen trockener Haut eine solche Formulierung nur bedingt geeignet.

Eine Aufgabe der vorliegenden Erfindung ist es daher, die geschilderten Nachteile durch Bereitstellung einer neuen Formulierung zu überwinden, welche jedoch die entzündungshemmende und analgetische Wirkung im gleichen Ausmaß wie die Gelform aufweist.

Überraschenderweise ist es gelungen, eine Salben-Zubereitung zu finden, welche die geforderten Eigenschaften aufweist.

Gegenstand der vorliegenden Erfindung ist daher eine Salben-Formulierung enthaltend Etofenamat sowie ein Adjuvans.

Etofenamat stellt ein hochviskoses Öl dar, welches in Wasser nur in Spuren löslich ist. In einer Vielzahl von galenischen Hilfsstoffen besteht nur eine mangelnde Löslichkeit, finden Interaktionen, wie z.B. Hydrolyse, Umesterung, Veresterung etc. statt, tritt Entmischung, Phasentrennung etc. auf. Einige Formulierungen mit Etofenamat haben sich zwar als homogen und stabil erwiesen, wiesen jedoch eine im Vergleich zum bekannten Etofenamathaltigen Gel ungenügende Resorption auf. Diese Nachteile treten bei der erfindungsgemäßen Zubereitung nicht auf. In der erfindungsgemäßen Zusammensetzung wird Etofenamat hervorragend von der Haut aufgenommen.

Die erfindungsgemäße Salben-Formulierung enthält daher neben Etofenamat noch ein Adjuvans und gegebenenfalls Konistenzgeber und/oder Emulgatoren sowie weitere Hilfsstoffe, wie durchblutungsfördernde bzw. wärmende oder

angenehm riechende Zusätze.

Erfindungsgemäß wird als Adjuvans eines oder mehrere aus den Gruppen:

- Triglyceride und/oder
- Ester ein- und/oder mehrwertige Alkohole und/oder
- höhere Alkohole

eingesetzt.

Als Adjuvans aus der Gruppe Triglyceride wird vorzugsweise ein oder mehrere Triglyceride von mittelkettigen Carbonsäuren der Kettenlänge $C_6$ bis $C_{14}$ in gesättigter oder ungesättiger sowie verzweigter oder unverzweigter Form, besonders bevorzugt Neutralöle, wie z.B. Miglyol 810 oder Miglyol 812 oder Viscoleo eingesetzt. Unter Viscoleo wird ein Gemisch von gesättigten Triglyceriden mittlerer Kettenlänge und der Fettsäureverteilung 45 bis 60 % Caprylsäure, 35 bis 50 % Caprinsäure und 2 bis 10 % Laurinsäure, unter Miglyol ein Capryl-Caprin-Säuretriglycerid verstanden.

Unter Adjuvans aus der Gruppe Ester ein- und/oder mehrwertiger Alkohole wird vorzugsweise einer oder mehrere Ester ein- und/oder mehrwertiger Alkohole der Kettenlänge $C_1$ - $C_{18}$ mit Carbonsäurekomponenten der Kettenlänge $C_6$ - $C_{18}$, besonders bevorzugt Propylenglykoldiester, Ethyloleat, Isopropylmyristat, -stearat, -palmitat, Diisopropyladipat, Diaethylsebacat, Ölsäureoleylsäureester,

Laurinsäurehexylester, Isooctylstearat, ganz besonders bevorzugt Diisopropyladipat und/oder Isopropylmyristat verstanden.

Unter Adjuvans aus der Gruppe höhere Alkohole wird vorzugsweise Oleylalkohol und/oder 2-Octyl-dodecanol, besonders bevorzugt 2-Octyl-dodecanol alleine verstanden.

Erfindungsgemäß kann Etofenamat in den Formulierungen mit einem oder mehreren Adjuvantien aus allen drei oder nur zwei oder nur einer Gruppe gemischt vorliegen.

Ausgehend von diesen Etofenamat-Adjuvans-Gemischen bzw. Lösungen können verschiedene Salben der Konsistenz flüssig bis halbfest hergestellt werden.

Erfindungsgemäß ist es notwendig, daß die aus Wirkstoff und Adjuvans bestehende Salbe als Wirkstoff ca. 5 bis ca. 40 Gew.-% Etofenamat und ca. 3 bis ca. 95 Gew.-% Ajuvans oder wie oben angeführt Adjuvans-Mischungen enthält. Bevorzugt enthält die Mischung 5 bis 15 Gew.-% Etofenamat, besonders bevorzugt 6 bis 12 Gew.-%.

Ferner kann die Zubereitung noch einen oder mehrere Konsistenzgeber und/oder einen oder mehrere Emulgatoren und/oder durchblutungsfördernde bzw. wärmende Zusätze und/oder weitere Hilfsstoffe, wie z.B. angenehm riechende Stoffe und/oder auch weitere Wirkstoffe, enthalten.

In vielen Fällen sind auch Konsistenzgeber und Emulgatoren identisch, eine strikte Trennung ist daher nicht möglich.

Als Konsistenzgeber kann einer oder mehrere aus der Gruppe Fettalkohole der Kettenlänge $C_{10}$ bis $C_{30}$, wie z.B. Stearylalkohol, Kohlenwasserstoffe, wie Vaseline, Paraffine, Hartparaffine, Wachse wie Bienen- oder Carnaubawachs eingesetzt werden. Außerdem können noch Mono-, Di- und Triglyceride wie Glycerinmonostearat, Glycerindistearat, Metallseifen wie Al-stearat, Aerosil, Polyglykole wie Polyethylenglykole, Polypropylenglykole verwendet werden.

Als Emulgator kann einer oder mehrere aus der Gruppe der ionogenen oder nichtionogenen Wasser-in-Öl und Öl-in-Wasser-Emulgatoren eingesetzt werden.

Bevorzugt werden Salze höherer Fett- und Gallensäuren wie Triethanolaminstearat, Alkylsulfate wie Na-Laurylsulfat, Alkylsulfonate wie Na-Cetylsulfonat, höhere Fettalkohole wie Cetylalkohol, Laurylalkohol und Stearylalkohol, Sterinalkohole wie Cholesterin, Fettsäureester mehrwertiger Alkohole und Polyole wie Ethylenmonostearat, Glycerinmonooleat, Sorbitanmonolaurat, Sorbitantrioleat, Polyoxyethylen-(20)-sorbitanmonolaurat, Polyoxyethylen-Sorbit-Hexaoleat, Fettalkoholäther wie Polyoxyethylenlaurylether, Polyoxyethylenoleylether, Fettsäureester der Saccharose wie Saccharosedistearat, Lecithin und Derivate, Betaine und Sulfobetaine wie Fettsäureamidoalkylbetain und/oder Polyoxyethylenpolyoxypropylen-Polymere

wie Pluronics eingesetzt.

Als durchblutungsfördernde oder wärmende Zusätze können z.B. Benzylnicotinat, Salicylsäureester, z.B. Methylsalicylat, etherische Öle, Capsaicin, Capsicumextrakt oder Nonylsäurevanillylamid eingesetzt werden.

Als Geruchsstoffe können die üblichen pharmakologisch unbedenklichen Stoffe eingesetzt werden, sofern sie sich mit dem Wirkstoff und Adjuvans vertragen.

Die Menge der Konsistenzgeber und/oder Emulgatoren in der Salben-Formulierung beträgt neben Etofenamat und Adjuvans bis zu ca. 70 Gew.-%, bevorzugt 5 bis 25 Gew.%.

Besonders bevorzugt sind Zubereitungen die 5 bis 40 Gew.% Etofenamat und 3 bis 95 Gew.% Adjuvans enthalten.

Besonders bevorzugt sind ferner die Zubereitungen deren Zusammensetzung aus den Beispielen hervorgeht.

Erfindungsgemäß werden die erfindungsgemäßen entzündungshemmenden, analgetischen Zubereitungen hergestellt, indem das in einem geeigneten Lösungsmittel gelöste Etofenamat mit den gegebenenfalls übrigen aufgeschmolzenen Fett-/Wachs-Emulgator-Bestandteilen gemischt und anschließend einer Homogenisierung beispielsweise mittels einer Rotor-Stator-Einrichtung unterworfen wird.

Die Homogenisierungstemperatur ist von der jeweiligen Zubereitung abhängig und liegt im allgemeinen zwischen ca. 40°C und ca. 60°C.

In der erfindungsgemäßen Zubereitung des Etofenamats ist der Wirkstoff in stabiler Form enthalten und vermag ohne die Schlepperfunktion des Isopropanols, welches im Gel enthalten ist, die Haut zu penetrieren. Vergleichsstudien zu Etofenamat-Gel ergaben bioäquivalente Resorption, was durch annähernd gleiche Resorptionsgeschwindigkeit, Zeit maximalen Plamaspiegels, maximaler Plamaspiegelkonzentration, Elimination aus dem Plasma, dem Flächeninhalt unter den Plamaspiegelkurven (AUC) sowie der renalen Elimination beim Menschen belegt werden konnte.

Die Gleichwertigkeit der Resorption ergibt sich auch aus klinischen Prüfungen, in denen die Wirksamkeit im Doppelbild cross over-Versuch gegen 1-(p-Chlorbenzoyl)-5-methoxy-2-methylindol-3-essigsäure enthaltende Salbe sowie gegen Salicylat-haltige Salben verglichen wurde und bei denen die erfindungsgemäße Zubereitung z.B. bei Lumbago hinsichtlich Schober-Distanz, Spontan-, Bewegungs- und Druckschmerz gegenüber dem ersten Vergleichspräparat signifikant ( $p < 0,05$) besser wirkte und sich z.B. bei akuter Gonarthrose bei den Zielgrößen Gelenkbeweglichkeit, Gelenkumfang, Spontan- und Bewegungsschmerz gegenüber dem zweiten Vergleichspräparat signifikant wirksamer erwies.

Die vorliegende Erfindung soll durch die nachfolgenden Beispiele noch weiter veranschaulicht werden.

Tabelle

| Beispiel | Etofenamat (mg) | Adjuvans I (mg) | Adjuvans II (mg) | Emulgator I (mg) | Konsistenzgeber (mg) | Aerosil (mg) |
|---|---|---|---|---|---|---|
| 1 | 50 | Diisopropyladipat 820 | - | Cremophor EL 50 | - | 80 |
| 2 | 200 | " 670 | - | " 50 | - | 80 |
| 3 | 50 | Isopropylmyristat 900 | - | " 50 | - | - |
| 4 | 200 | " 750 | - | " 50 | - | - |
| 5 | 50 | Miglyol 840 900 | - | Tween 40 50 | - | - |
| 6 | 200 | " 670 | - | " 50 | - | 80 |
| 7 | 50 | Miglyol 812 820 | - | Tween 60 50 | - | 80 |
| 8 | 200 | " 750 | - | " 50 | - | - |
| 9 | 50 | Oleylalkohol 450 | Diisopropylstearat 450 | Cremophor EL 50 | - | - |
| 10 | 100 | Isopropylmyristat 770 | - | Tween 40 50 | - | 80 |
| 11 | 100 | " 500 | Diisopropyladipat 260 | Tween 60 50 | - 80 | |

Tabelle

| Beispiel | Etofenamat (mg) | Adjuvans I (mg) | Adjuvans II (mg) | Emulgator I (mg) | Konsistenzgeber (mg) | Aerosil (mg) | Cetyl-alkohol | PEG 400 | Nicotin-säure benzylester |
|---|---|---|---|---|---|---|---|---|---|
| 12 | 50 | Diisopropyl-adipat 100 | - | Myrj 59 50 | Wollwachsalkohol-salbe DAB 8 780 | - | 20 | — | — |
| 13 | 200 | Diisopropyl-adipat 150 | - | Myrj 59 50 | Wollwachsalkohol-salbe DAB 8 570 | - | 30 | — | — |
| 14 | 50 | Isopropyl-myristat 100 | - | Myrj 59 50 | Softisan 378 770 | 30 | - | — | — |
| 15 | 200 | Isopropyl-myristat 120 | - | Myrj 59 50 | Softisan 378 615 | 35 | - | — | — |
| 16 | 200 | Isopropyl-myristat 120 | - | Myrj 59 50 | PEG 4000 400 | - | - | 230 | — |
| 17 | 50 | Miglyol 810 100 | - | Myrj 59 50 | Wollwachsalkohol-salbe DAB 8 780 | - | 20 | — | — |
| 18 | 200 | Miglyol 810 150 | - | Myrj 59 50 | Wollwachsalkohol-salbe DAB 8 545 | 25 | 30 | — | — |
| 19 | 50 | Miglyol 840 100 | - | Myrj 59 50 | Wollwachsalkohol-salbe DAB 8 780 | - | 20 | — | — |
| 20 | 200 | Miglyol 840 150 | - | Myrj 59 50 | Wollwachsalholol-salbe DAB 8 545 | 25 | 30 | — | — |
| 21 | 50 | Isopropyl-myristat 520 | - | Myrj 59 50 | Cutina MD 380 | - | - | — | — |
| 22 | 200 | Isopropyl-myristat 450 | - | Myrj 59 50 | Cutina MD 300 | - | - | — | — |
| 23 | 100 | Diisopropyl-adipat 840 | - | Myrj 59 | - | - | - | — | 10 |

| | |
|---|---|
| Miglyol 840 | = Propylenglykoldiester der Capryl-Caprinsäure |
| Myrj 59 | = Polyoxyethylenfettsäureester |
| Tween 40 | = Polyoxyethylen(20)sorbitanmonopalmitat |
| Tween 60 | = Polyoxyethylen(20)sorbitanmonopalmitat |
| Cremophor EI | = Glycerin-Poylethylenglykolricinoleat |
| Cutina MD/GMS | = Gemisch von Mono- und Diglyceriden der Palmitin- und Stearinsäure |
| Softisan378 | = Triglyceride der Stearin-, Caprin- und Caprylsäure. |

Patentansprüche:

1. Zubereitung enthaltend Etofenamat und ein oder mehrere Adjuvantien sowie gegebenenfalls Konsistenzgeber und/oder Emulgatoren und/oder weitere Hilfsstoffe.

2. Zubereitung nach Anspruch 1 enthaltend als Adjuvans eines oder mehrere aus den Gruppen:
- Triglyceride,
- Ester ein- und/oder mehrwertiger Alkohole,
- höhere Alkohole.

3. Zubereitung nach Anspruch 1 oder 2 enthaltend als Adjuvans ein oder mehrere Triglyceride von mittelkettigen Carbonsäuren der Kettenlänge $C_6$ bis $C_{14}$ in gesättigter oder ungesättigter sowie verzweigter oder unverzweigter Form, bevorzugt Neutralöle und/oder Viscoleo.

4. Zubereitung nach einem oder mehreren der Ansprüche 1 bis 3 enthaltend einen oder mehrere Ester ein- und/oder mehrwertiger Alkohole der Kettenlänge $C_2$ bis $C_{18}$ mit Carbonsäurekomponenten der Kettenlänge $C_6$ bis $C_{18}$, besonders bevorzugt Propylenglykoldiester, Ethyloleat, Isopropylmyristat, -stearat, -palmitat, Diisopropyladipat, Diethylsebacat, Ölsäureoleylester, besonders bevorzugt Diisopropyladipat und/oder Isopropylmyristat.

5. Zubereitung nach einem oder mehreren der Ansprüche 1 bis 3, enthaltend Oleylalkohol und/oder 2-Octyldodecanol.

TP 66

6. Zubereitung nach einem oder mehreren der Ansprüche 1 bis 5, enthaltend 5 bis 40 Gew.-% Etofenamat und 3 bis 95 Gew.-% Adjuvans.

7. Verfahren zur Herstellung der Zubereitungen nach einem oder mehreren der Ansprüche 1 bis 6, dadurch gekennzeichnet, daß man in einem geeigneten Lösungsmittel gelöstes Etofenamat mit den gegebenenfalls übrigen Bestandteilen mischt und anschließend die Mischung homogenisiert.

8. Verfahren nach Anspruch 7, dadurch gekennzeichnet, daß die Homogenisierungstemperatur zwischen ca. 40°C und ca. 60°C liegt.

9. Verwendung der Zubereitung nach einem oder mehreren der Ansprüche 1 bis 6 als topisches Analgetikum.

10. Verwendung der Zubereitung nach einem oder mehreren der Ansprüche 1 bis 6 als topisches Antiphlogistikum für die human- und veterinärmedizinische Anwendung.

TP 66